# EUROPEAN PATENT APPLICATION

(11) **EP 2 286 775 A1**
(43) Date of publication of application: **23.02.2011**
(21) Application number: 09758328.0
(22) Date of filing: 02.06.2009
(51) Int. Cl.: A61F 13/15, A61F 5/44, A61F 13/49, A61F 13/494

(54) **ABSORBENT ARTICLE**

(30) Priority: 03.06.2008 JP 2008146173
(71) Applicant: Unicharm Corporation, Ehime 799-0111 (JP)
(72) Inventor: NAKAJIMA, Kaiyo, Kanonji-shi Kagawa 769-1602 (JP); MINATO, Hironao, Kanonji-shi Kagawa 769-1602 (JP)
(74) Representative: Stark, Gordon Drummond
(86) International application number: PCT/JP2009/060089
(87) International publication number: WO 2009/148061

(57) **Abstract**

An absorbent article includes a chassis 2, a liquid-absorbent structure 15 placed the chassis and a separate sheet 18 adapted to be spaced from the chassis 2. Opposite lateral regions 21, 22 of the separate sheet are joined to an inner sheet 6 via first joint zones 30, 31. First joint zones 30, 31 continuously extend in a longitudinal direction Y and substantially overlap the absorbent structure 15 as viewed in a thickness direction. Leak-barrier cuffs 35, 36 provided on the side of the separate sheet are joined to the inner sheet 6 via second joint zones 41, 42 lying outside the separate sheet as viewed in a transverse direction X, joined to the separate sheet 18 via third joint zones 50, and joined to the separate sheet via fourth joint zones 51.

## Description

### TECHNICAL FIELD

The present invention relates to absorbent articles and more particularly to absorbent articles such as disposable diapers, toilet-training pants or incontinent briefs.

### RELATED ART

Disposable diaper including a sheet formed with through-holes for passage of urine and feces, respectively, are known. For example, one of the diapers is disclosed in the disclosure of Japanese Patent Application Laid-Open Publication No.09-510385 (PATENT DOCUMENT 1). According to the disclosure of this PATENT DOCUMENT 1, the diaper includes a topsheet, a backsheet and a floating sheet provided on the inner side of the topsheet. The floating sheet is formed with front and rear through-holes for passage of urine and feces. In a longitudinal direction along these through-holes, the floating sheet is provided with floating sheet biasing elastic members attached thereto under tension so that the floating sheet may be spaced apart from a chassis and brought into contact with the wearer's skin. Urination and defecation occur through the front and rear through-holes of the floating sheet spaced from the chassis and thereby the wearer's skin is protected from direct contamination with body waste such as feces.

Along outer side edges of the floating sheet as viewed in the transverse direction, leg elastic members extending in the longitudinal direction are sandwiched under tension between the top- and backsheets. Contraction of the leg elastic members puts the opposite side edges of the floating sheet in close contact around the wearer's legs and prevents leakage of body waste such as urine.
[PATENT DOCUMENT 1] JP 09-510385 A

### DISCLOSURE OF THE INVENTION

### PROBLEM TO BE SOLVED BY THE INVENTION

With the diaper as has been described above put on the wearer, the opposite side edges of the top- and backsheets as well as the leg elastic members inevitably follow movement of the wearer's legs. Movement of the leg elastic members causes the floating sheet defined inside these elastic members as viewed in the transverse direction to move. Eventually the floating sheet may be spaced apart from the wearer's skin and/or through-holes of the floating sheet may be deformed. As a result, the respective through-holes may be misaligned with the associated excretion organs of the wearer and body waste may be discharged directly on the floating sheet. The body waste such as feces discharged on the floating sheet may come in contact with wearer's skin and cause various types of skin troubles.

It is an object of the present invention to provide an absorbent article improved to minimize the possibility that guide passages formed in a separate sheet (floating sheet) adapted to be spaced apart from a chassis might be misaligned with the associated excretion organs of the wearer.

### MEASURE TO SOLVE THE PROBLEM

According to the present invention, there is provided an improvement in an absorbent article comprising a chassis having a longitudinal direction and a transverse direction, sides facing the skin of a wearer and a garment worn by the wearer, respectively, a front waist region, a rear waist region and a crotch region and a liquid-absorbent structure placed at least in the crotch region, a separate sheet provided on the side facing the skin of the wearer of the chassis so that the separate sheet may be spaced apart from the crotch region, and leg elastic members attached under tension in the longitudinal direction so as to extend across the crotch region in the longitudinal direction.

According to the present invention, the separate sheet comprises a pair of lateral regions opposed to each other in the transverse direction and extending in the longitudinal direction, a middle region lying between the lateral regions so as to connect the lateral zones to each other, guide passages adapted to ensure fluid-communication between the side facing the skin of the wearer and the side of the chassis, and joint zones used to join the lateral regions to the chassis, wherein the separate sheet is elasticized in the longitudinal direction at least in the crotch region and the joint zones are formed so as to overlap the liquid-absorbent structure. The leg elastic members are provided outside the liquid-absorbent structure as viewed in the transverse direction. A void space is formed between the separate sheet and the liquid-absorbent structure and at least the middle region comes in contact with the skin of the wearer as the liquid-absorbent structure is curved so that front and rear ends thereof opposed in the longitudinal direction and extending in the transverse direction face each other in the absorbent article put on the wearer.

According to one preferred embodiment, the joint zones are arranged so as to extend in the longitudinal direction at least in the crotch region.

According to another preferred embodiment, the lateral regions of the separate sheet are formed with two-ply folded zones by folding back the lateral regions toward the side of the chassis along fold lines extending in the longitudinal direction and the joint zones are formed in these two-ply folded zones.

According to still another preferred embodiment, the joint zones include fluid-communicating pores for fluid-communication between the interior and the exterior of the joint zones as viewed in the transverse direction in regions aside from a transverse center line bisecting a dimension of the chassis in the longitudinal direction toward the front waist region.

According to yet another preferred embodiment, the chassis further comprises a pair of leak-barrier cuffs extending in the longitudinal direction on the side facing the skin of the wearer, each of these leak-barrier cuffs includes a proximal side edge fixed to the chassis and a free side edge adapted to be spaced from the chassis, and the proximal side edge is provided with the leg elastic members attached thereto under tension.

According to further another preferred embodiment, the leg elastic members extend substantially in parallel to the lateral regions of the separate sheet.

According to still further another preferred embodiment, the leg elastic members extend from respective boundary points between the front waist region and the crotch region to respective boundary points between the rear waist region and the crotch region, respective opposite ends of the leg elastic members lie outside the liquid-absorbent structure as viewed in the transverse direction, and the joint zones overlap the liquid-absorbent structure at positions corresponding to the respective opposite ends.

According to still further another preferred embodiment, the separate sheet further comprises a pair of separate sheet elastic members, and the separate sheet elastic members are attached under tension at least along lateral edges of the guide passages.

### EFFECT OF THE INVENTION

The separate sheet has a pair of lateral regions opposed to each other in the transverse direction and extending in the longitudinal direction are joined to the chassis by the intermediary of the joint zones and these joint zones are arranged so as to overlap the liquid-absorbent structure. This means that the separate sheet is joined to the liquid-absorbent structure having a relatively high rigidity. The leg elastic members are arranged so as to extend outside the liquid-absorbent structure as viewed in the transverse direction. With this a unique arrangement, even if the leg elastic members tend to follow movement of the legs of the wearer, a potential movement of the leg elastic members is restricted by the liquid-absorbent structure and any movement of the leg elastic members is effectively prevented from being transmitted to the separate sheet. Undesirable displacement is restricted and, in consequence, the guide passages formed in the separate sheet should not be misaligned with the associated excretion organs of the wearer and body waste should not be discharged on the side of the separate sheet facing the skin of the wearer.

The arrangement of the joint zones so as to extend in said longitudinal direction at least in said crotch region makes it possible to restrict undesirable displacement of the separate sheet due to movement of the leg elastic members in the region which is significantly affected by movement of these elastic members due to movement of the legs of the wearer. In consequence, the guide passages formed in the separate sheet should not be misaligned with the associated excretion organs of the wearer can be further reliably restricted.

The lateral regions of the separate sheet are formed with two-ply folded zones by folding back the lateral regions toward the side of the chassis along fold lines extending in the longitudinal direction and the joint zones are formed in these two-ply folded zones. Such an arrangement makes it possible to enlarge a distance by which the separate sheet can be spaced apart from the chassis and thereby to minimize the possibility that movement of the leg elastic members might affect the separate sheet.

According to one of the preferred embodiments, the joint zones include fluid-communicating pores for fluid-communication between the interior and the exterior of the joint zones as viewed in the transverse direction in regions aside from a transverse center line bisecting a dimension of the chassis in the longitudinal direction toward the front waist region. Such a unique arrangement makes it possible to transfer a quantity of body waste, e.g., urine discharged inside the joint zones as viewed in the transverse direction toward the outside the joint zones. Thus body waste such as urine can be absorbed by the liquid-absorbent structure on both sides of the joint zones. In this way, body waste such as urine can be absorbed over a wide area and thereby leakage of body waste such as urine can be effectively prevented.

According to one of the preferred embodiments, the chassis further comprises a pair of leak-barrier cuffs extending in the longitudinal direction on the side facing the skin of the wearer and the proximal side edges are provided with the leg elastic members attached thereto. Such an arrangement makes it possible to attach the leg elastic members to a sheet provided independently of the sheet constituting the liquid-absorbent structure. In this way, the separate sheet can be further reliably protected from undesirable affection of the leg elastic members which are movable following movement of the legs of the wearer transmitted by the liquid-absorbent structure.

The leg elastic members arranged to extend substantially in parallel to the lateral regions of the separate sheet makes it possible to maintain a distance between the joint zones formed on the lateral regions and the leg elastic members substantially constant and thereby to maintain a potential affection of the leg elastic members upon the separate sheet substantially constant. In this way, locally increased affection of the leg elastic members can be avoided.

According to one of the preferred embodiments, the leg elastic members are formed by rubber tape and these leg elastic members extend from respective boundary points between the front waist region and the crotch region to respective boundary points between the rear waist region and the crotch region, i.e., extend over the full length of the crotch region in the longitudinal direction. The respective opposite ends of these leg elastic members lie outside the liquid-absorbent structure as viewed in the transverse direction and the joint zones overlap the liquid-absorbent structure at positions corresponding to the respective opposite ends. With such a unique arrangement, it is possible to minimize undesirable affection of the leg elastic members upon the joint zones in the crotch region in which the leg elastic members are most apt to move.

### BRIEF DESCRIPTION OF THE DRAWINGS

Fig. 1 is a perspective view of a diaper according to a first embodiment.
Fig. 2 is a plan view showing this diaper as has been flatly developed.
Fig. 3 is an exploded perspective view of the diaper.
Fig. 4 is a sectional view taken along the line IV-IV in Fig. 2.
Fig. 5 is a sectional view taken along the line V-V in Fig. 2.
Fig. 6 is a sectional view taken along the line VI-VI in Fig. 2.
Fig. 7 is a schematic diagram corresponding to Fig. 2.
Fig. 8 is a sectional view taken along the line VIII-VIII in Fig. 1.
Fig. 9 is a view similar to Fig. 7, illustrating a diaper according to the second embodiment.

### IDENTIFICATION OF REFERENCE NUMERALS USED IN THE DRAWINGS

- 1: diaper (absorbent article)
- 2: chassis
- 3: front waist region
- 4: rear waist region
- 5: crotch region
- 15: liquid-absorbent structure
- 15a: front end
- 15b: rear end
- 18: separate sheet
- 21: lateral region
- 22: lateral region
- 23: middle region
- 24: front guide passage
- 25: rear guide passage
- 26: fold line
- 27: fold line
- 28: folded region
- 29: folded region
- 30: first joint zone (joint zone)
- 31: first joint zone (joint zone)
- 33: separate sheet elastic member
- 34: separate sheet elastic member
- 35: leak-barrier cuff
- 36: leak-barrier cuff
- 37: proximal side edge
- 38: proximal side edge
- 39: free side edge
- 40: free side edge
- 45: leg elastic tape
- 46: leg elastic tape
- 48: fluid-communicating pore
- 49: fluid-communicating pore

### DETAILED DESCRIPTION OF THE PREFERRED EMBODIMENTS

Details of an absorbent article according to the present invention will be more fully understood from the description of a disposable diaper, one embodiment of the absorbent article, given hereunder with reference to the accompanying drawings.

### <First Embodiment>

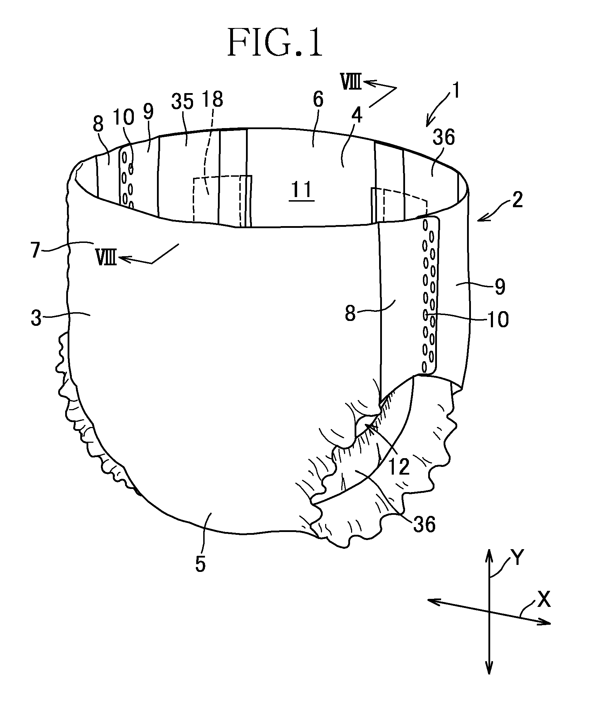

Figs. 1 through 8 illustrate a first embodiment of the invention wherein Fig. 1 is shows a diaper 1 with waist- and leg-openings opened. As illustrated, the diaper 1 according to this embodiment is of so-called pullon-pant type. The diaper 1 is pant-shaped by a chassis 2 comprising a front waist region 3, a rear waist region 4 and a crotch region 5 extending between these two waist regions 3, 4. A direction defined from the front waist region 3 across the crotch region 5 to the rear waist region is referred herein to as a longitudinal direction Y and a direction orthogonal thereto is referred herein to as a transverse direction X.

The chassis 2 comprises an inner sheet 6 defining an inner side facing the skin of the wearer, an outer sheet 7 defining an outer side facing a garment worn by the wearer and front and rear flaps 8, 9 attached to the inner sheet 6 and the outer sheet 7 in the front and rear waist regions 3, 4, respectively. The front and rear flaps 8, 9 are formed of elasticized sheets adapted to be elastically contractible at least in the transverse direction X. The inner side facing the skin of the wearer of the front flaps 8 is placed upon and intermittently joined to the outer side facing the skin of the wearer of the rear flaps 9 at joints 10 to obtain the pant-shaped chassis 2. Thereupon, a waist-opening 11 and a pair of leg-openings 12 each having a circumferential periphery defined by the front and rear waist regions 3, 4.

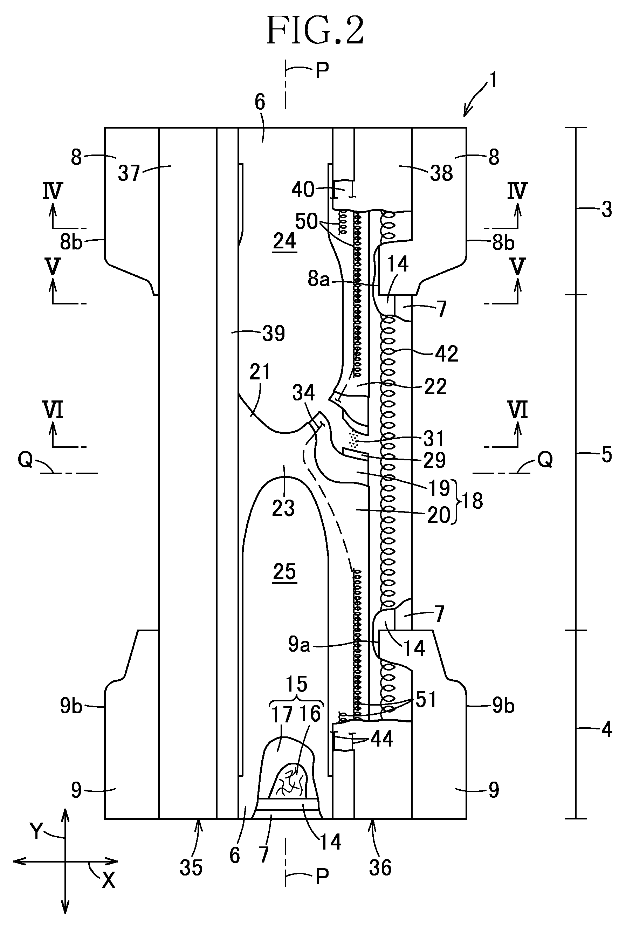

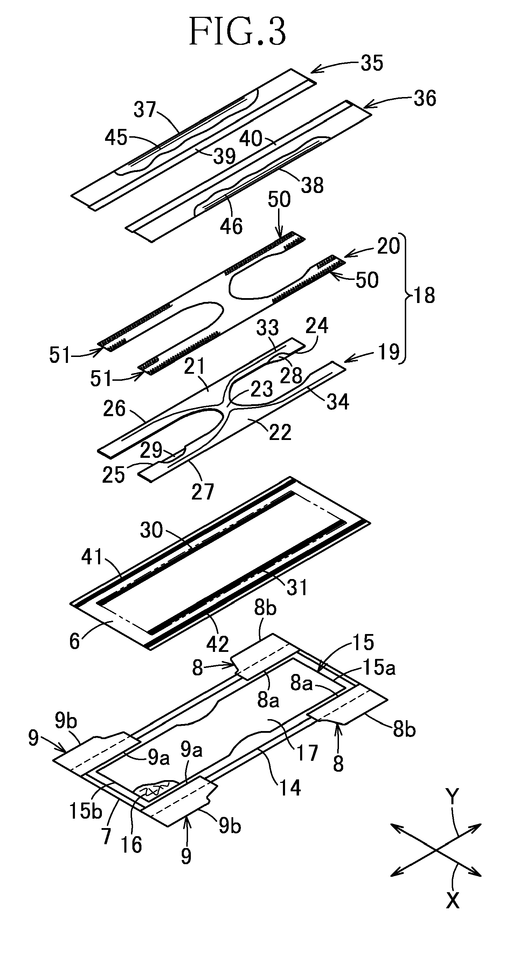

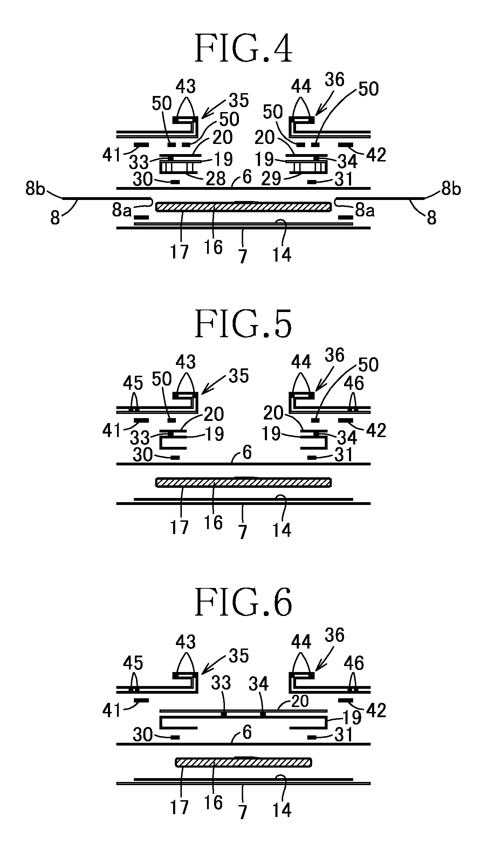

Fig. 2 is a plan view showing the diaper 1 developed in the longitudinal direction Y as well as in the transverse direction X, after the respective pairs of front and rear flaps 8, 9 have been unjoined each other at the joints 10, until the front and rear waist regions 3, 4 and the crotch region 5 are flattened in a coplanar relationship. In Fig. 2, the diaper 1 is partially broken away for convenience of illustration. Fig. 3 is an exploded perspective view of the diaper 1 corresponding to Fig. 2 as partially broken away herein also for convenience of illustration and on the assumption that each of elastic members has a contractile force thereof inactivated so that the diaper 1 remains planar. The diaper 1 has a longitudinal center line P-P bisecting its dimension in the transverse direction X and a transverse center line Q-Q bisecting its dimension in the longitudinal direction Y. The diaper 1 is bilaterally symmetric about the longitudinal center line P-P. Figs. 4, 5 and 6 are sectional views taken along lines IV-IV, V-V and Vi-VI, respectively, in Fig. 2.

As will be apparent from Fig. 3, both the inner sheet 6 and the outer sheet 7 are substantially the same in shape as well as in size and formed by fibrous nonwoven fabrics, each extending in the longitudinal direction to have a substantially rectangular shape. On the side of the outer sheet 7 facing the skin of the wearer there is provided a liquid-impervious leak-barrier sheet 14 and on the side of the leak-barrier sheet 14 facing the skin of the wearer there is provided a liquid-absorbent structure 15. The inner sheet 6 and the outer sheet 7 are formed of an air-permeable and hydrophobic nonwoven fabric and joined to each other by the intermediary of the lead-barrier sheet 14 and the liquid-absorbent structure 15.

The liquid-absorbent structure 15 includes front and rear ends 15a, 15b opposed to each other in the longitudinal direction Y and extending in the transverse direction X. The liquid-absorbent structure 15 comprises a liquid-absorbent core 16 and a liquid-absorbent dispersant sheet 17 wrapping the liquid-absorbent core 16 and extends across the crotch region 5 toward or into the front and rear waist regions 3, 4.

In the front and rear waist regions 3, 4, the front and rear flaps 8, 9 have inner side edges 8a, 9a, respectively, attached to the inner sheet 6 and the outer sheet 7 and outer side edges 8b, 9b extending outward in the transverse direction X. On the side of the inner sheet 6 facing the skin of the wearer there is provided a separate sheet 18 comprising a first sheet 19 lying on the side of the inner sheet 6 and a second sheet 20 laminated with the side of the first sheet 19 facing the wearer's skin. The separate sheet 18 comprising these first and second sheets 19, 20 has a pair of lateral regions 21, 22 opposed to each other in the transverse direction X and a middle region 23 connecting the lateral regions 21, 22 wherein the middle region 23 lies in the crotch region 5. These lateral regions 21, 22 and middle region 23 cooperate together to define front and rear guide passages 24, 25. The front guide passage 24 gradually broadens from the middle region 23 toward the front waist region 3 so as to form a substantially U-shaped through-hole while the rear guide passage 25 gradually broadens from the middle region 23 toward the rear waist region 4 so as to form a substantially U-shaped through-hole.

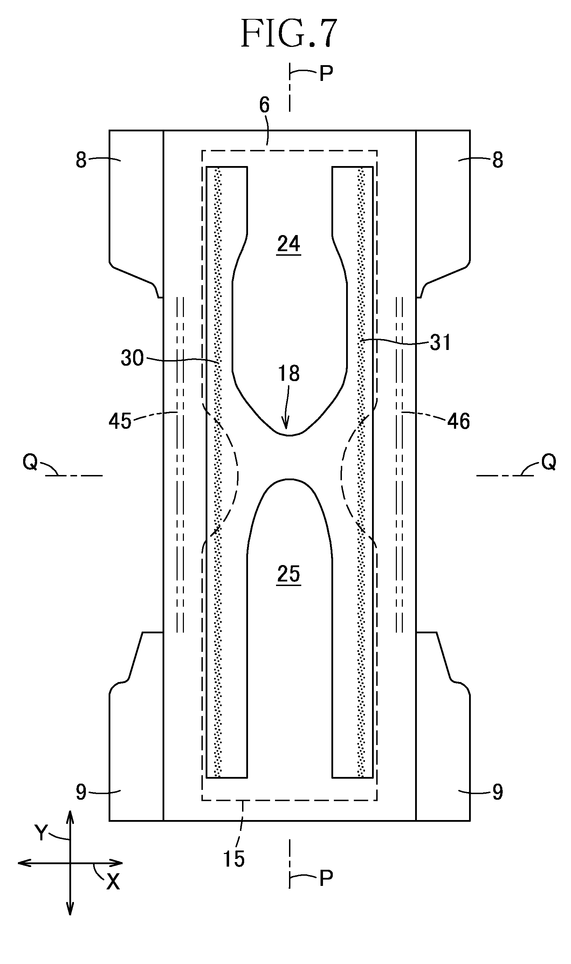

The first sheet 19 is formed in the lateral regions 21, 22 with fold lines 26, 27 extending in the longitudinal direction Y and the lateral regions 21, 22 are folded back toward the side of the inner sheet 6 along these fold lines 26, 27 so as to form two-ply folded zones 28, 29. These two-ply folded zones 28, 29 are joined to the inner sheet 6 to form a pair of first joint zones 30, 31. Fig. 7 is a schematic diagram corresponding to Fig. 2 but illustrates the diaper 1 devoid of a pair of leak-barrier cuffs 35, 36 in order to clarify a position relationship between the first joint zones 30, 31. As illustrated, the first joint zones 30, 31 continuously extend from the front end to the rear end of the separate sheet 18 in the longitudinal direction Y and substantially overlap the liquid-absorbent structure 15 as viewed in a thickness direction.

A pair of separate sheet elastic members 33, 34 is attached under tension between the first sheets 19, 20. Specifically, these separate sheet elastic members 33, 34 extend along the front and rear guide passages 24, 25 so that these elastic members 33, 34 are curved toward the longitudinal center line P-P in the middle region 32 of the separate sheet 18. The separate sheet elastic members 33, 34 are used to exert a contractile force in the longitudinal direction Y to the separate sheet 18 and therefore it will not be essential to use these elastic members 33, 34, for example, when the separate sheet 18 is formed of a sheet elasticized in the longitudinal direction Y.

On the side of the second sheet 20 constituting the separate sheet 18 facing the skin of the wearer, there is provided a pair of leak-barrier cuffs 35, 36. The leak-barrier cuffs 35, 36 are formed of a pair of two-ply sheets spaced apart from each other in the transverse direction X and extending in the longitudinal direction Y. The leak-barrier cuffs 35, 36 extend outside the separate sheet 18 and respectively include proximal side edges 37, 38 joined to the inner sheet 6 and free side edges 39, 40 lying on the side of the separate sheet 18 facing the skin of the wearer and spaced apart from the separate sheet 18. The proximal side edges 37, 38 extend along the respective outer edges of the leak-barrier cuffs 35, 36 as viewed in the transverse direction X and joined to the inner sheet 6 by the intermediary of second joint zones 41, 42 extending in the longitudinal direction Y. The leak-barrier cuffs 35, 36 are joined by the intermediary of third joint zones 50 in the front waist region 3 and joined to the separate sheet 18 by the intermediary of fourth joint zones 51 in the rear waist region 4.

The respective free side edges 39, 40 of the leak-barrier cuffs 35, 36 extend inside as viewed in the transverse direction X with respect to the proximal side edges 37, 38 and are not joined to the inner sheet 6 and the separate sheet 18 at least in the crotch region 5. Consequentially, the free side edges 39, 40 not joined to the inner sheet 6 can be spaced apart from the inner sheet 6 about the proximal side edges 37, 38 joined to the inner sheet 6.
The respective free side edges 39, 40 are folded outward in the transverse direction X and provided with cuff elastic members 43, 44 attached thereto under tension in the longitudinal direction Y. Specifically, the cuff elastic members 43, 44 are sandwiched between the respective two-ply sheet regions and extend across the front and rear waist regions 3, 4 in the longitudinal direction Y.

A plurality of leg elastic tapes 45, 46 is attached under tension in the longitudinal direction Y to the respective proximal side edges 37, 38. These leg elastic members 45, 46 are attached to the proximal side edges 37, 38 so as to extend in the crotch region 5 in the longitudinal direction Y between the front and rear waist regions 8, 9. Specifically, these leg elastic members 45, 46 extend from boundary points between the front waist region 3 and the crotch region 5 to boundary points between the rear waist region 4 and the crotch region 5. The proximal side edges 37, 38 are joined to the inner sheet 6 outside the liquid-absorbent structure 15 as viewed in the transverse direction X to prevent the proximal side edges 37, 38 from overlapping the liquid-absorbent structure 15. It should be appreciated here that the leg elastic members 45, 46 may partially overlap the liquid-absorbent structure 15 so far as at least respective pairs of opposite ends of these elastic members 45, 46 lying on the respective boundary points between the crotch region 5 and the front and rear waist regions 3, 4 do not overlap the absorbent structure 15. The leg elastic members 45, 46 are not limited to the rubber tapes but may be exploited in the form of thread- or strand-like or the other rubber members.

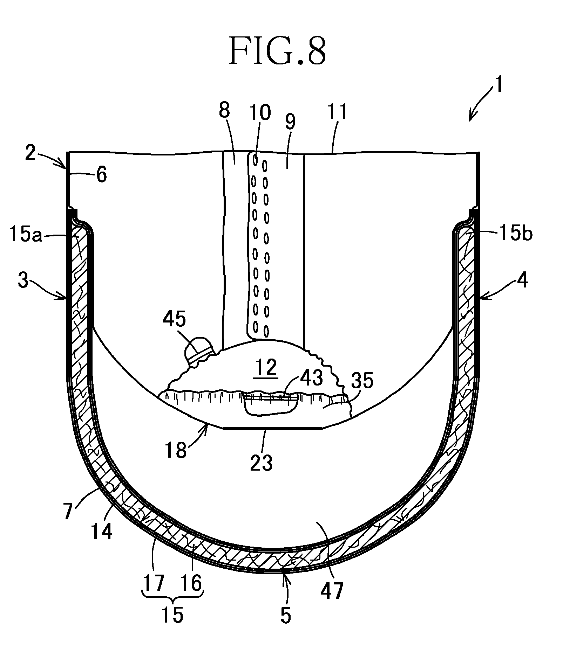

Fig. 8 is a sectional view taken along the line VIII-VIII in Fig. 1. As will be apparent from Fig. 8, when the diaper 1 constructed as has been described above is put on the wearer with the front and rear flaps 8, 9 joined together, the liquid-absorbent structure 15 bows with the front and rear ends 15a, 15b thereof opposed to each other and the leak-barrier cuffs 35, 36 are spaced apart upward from the topsheet 6 under contractile force of the cuff elastic members 43, 44 so as to form walls serving to retain bodily fluids such as urine within the diaper 1. At the same time, the separate sheet 18 is spaced apart from the liquid-absorbent structure 15 under contractile force of the separate sheet elastic members 33, 34 to form a void space 47 between the separate sheet 18 and the liquid-absorbent structure 15. The middle region 23 of the separate sheet 18 defined in the crotch region 5 is brought into contact with the crotch of the wearer as the separate sheet 18 is spaced apart upward from the liquid-absorbent structure 15. Thereupon the external genital of the wearer is aligned with the front guide passage 24, assuring proper urination through the front guide passage 24 into the void space 47. In a similar manner, the anus of the wearer is aligned with the rear guide passage 25, assuring proper defecation through the rear guide passage 25 into the void space 47. In this way, the skin of the wearer is reliably protected from soil with body waste and from skin troubles due to such soil with body waste can be reliably prevented.

With the diaper 1 put on the wearer, the front and rear waist regions 3, 4 are elasticized in the transverse direction X under contractile force of the front and rear flaps 8, 9, respectively, to maintain the front and rear waist regions 3, 4 in close contact with the skin of the wearer and thereby to prevent body waste such as urine from leaking upward beyond the waist regions. The crotch region 5 is elasticized in the longitudinal direction Y under contractile force of the leg elastic members 45, 46 to maintain the inner sheet 6 and the outer sheet 7 in close contact with the skin of the wearer and thereby to prevent body waste such as urine from leaking beyond the peripheral edges of the leg-openings.

According to this embodiment, the first joint zones 30, 31 are arranged so as to overlap the liquid-absorbent structure 15 and the leg elastic members 45, 46 are arranged outside the liquid-absorbent structure 15 as viewed in the transverse direction X so as not to overlap the liquid-absorbent structure 15. The liquid-absorbent structure 15 contains the liquid-absorbent core 16 and has a correspondingly higher rigidity in comparison with the other sheets. With such a unique arrangement, a potential displacement of the leg elastic members 45, 46 is restricted by the liquid-absorbent structure 15 even if the leg elastic members 45, 46 move as the wearer moves, and consequently such movement is not transmitted to the separate sheet 18. Thus the separate sheet 18 should not be undesirably moved relative to the wearer and the front and rear guide passages 24, 25 would be not out of the desired alignment with the external genital and anus of the wearer, respectively. Without the possibility of undesirable displacement, the side of the separate sheet 18 facing the skin of the wearer should not be soiled with body waste and thus skin troubles can be reliably prevented.

According to this embodiment, the leg elastic members 45, 46 are spaced apart from the first joint zones 30, 31, respectively, at least by a distance of approximately 10 mm or longer in the transverse direction X. By setting this distance to 10 mm or longer, potential movement of the leg elastic members 45, 46 can be further reliably prevented from being transmitted to the separate sheet 18.
While the first joint zones 30, 31 are arranged to extend from the front end 15a to the rear end 15b of the liquid-absorbent structure 15 as far as this embodiment is concerned, these joint zones 30, 31 may be provided at least in the crotch region 5 to meet the intended function. This is because while the front and rear waist regions of the chassis and the members directly associated therewith are relatively free from affection of movement of the legs of the wearer, the crotch region and the members directly associated therewith are readily displaced as the legs move and displacement of the leg elastic members 45, 46 in the crotch region 5 may cause the separate sheet 18 to be undesirably displaced.

The leak-barrier cuffs 35, 36 are joined to the inner sheet 6 by the intermediary of the second joint zones 41, 42 to form the proximal side edges 37, 38 about which the associated free side edges 39, 40 may be spaced apart upward from the separate sheet 18. The leak-barrier cuffs 35, 36 are joined to the separate sheet 18 by the intermediary of the third and fourth joint zones 50, 51 to ensure that the separate sheet 18 is pulled up toward the skin of the wearer as the leak-barrier cuffs 35, 36 are spaced apart upward from the liquid-absorbent structure 15.

While the lateral regions 21, 22 of the separate sheet 18 are formed with the two-ply folded zones 28, 29 according to this embodiment, it is not essential to form such two-ply folded zones 28, 29. Furthermore, these two-ply folded zones 28, 29 may be replaced by three-ply or four-ply folded zones. The number of ply may be increased to enlarge a distance by which the separate sheet 18 can be spaced from the liquid-absorbent structure 15 and thereby not only to alleviate a potential affection of movement of the leg elastic members 45, 46 but also to ensure the middle region 23 to be maintained in contact with the skin of the wearer.
While the leg elastic members 45, 46 are arranged only in the crotch region 5 according to this embodiment, these elastic members 45, 46 may be arranged also to extend across the crotch region 5 further into the front and rear waist regions 3, 4. By prolonging the leg elastic members 45, 46 in this manner, regions to be held in close contact with the thighs of the wearer may be enlarged and thereby an effect of urine leakage prevention can be correspondingly improved.

While the leg elastic members 45, 46 are attached to the proximal side edges 37, 38 of the respective leak-barrier cuffs 35, 36 according to this embodiment, it is possible to attach them between the leak-barrier cuffs 35, 36 and the inner sheet 6 or between the inner sheet 6 and the outer sheet 7. It should be noted that a distance between the leg elastic members 45, 46 and the separate sheet 18 may be enlarged to alleviate a possibility that displacement of the leg elastic members 45, 46 might affect the separate sheet 18. When the leg elastic members 45, 46 are sandwiched between the inner sheet 6 and the outer sheet 7, it is not essential to incorporate the leak-barrier cuffs 35, 36. While the leg elastic members 45, 46 are arranged so as to extend substantially in a rectilinear fashion in the longitudinal direction Y according to this embodiment, it is also possible, for example, to arrange these elastic members 45, 46 so as to be curved toward the longitudinal center line P-P in the crotch region 5. However, the leg elastic members 45, 46 may be preferably arranged so as to extend in the rectilinear fashion to maintain a constant distance from the opposite side edges of the separate sheet 18. The distance between the leg elastic members 45, 46 and the separate sheet 18 may be maintained constant to prevent the contractile force from being exerted directly upon the separate sheet 18 and thereby to prevent the contractile force of the leg elastic members 45, 46 from causing undesirable displacement of the separate sheet 18.

While the separate sheet 18 is formed with the front and rear guide passages 24, 25 according to this embodiment, it is also contemplated to for the separate sheet 18 with a single guide passage. However, it is preferred to form the separate sheet 18 with the front and rear guide passages 24, 25 in the front and rear waist regions 3, 4, respectively, and thereby to guide urine and feces separately through these two guide passages 24, 25 toward the liquid-absorbent structure 15. Preferably, a partition sheet is attached to the side of the middle region 23 facing the liquid-absorbent structure 15 so as to partition the void space 47 back and forth. By partitioning the void space 47 in this manner, urine and feces should not be mixed together within the void space 47.

The separate sheet 18 may be formed of a liquid-resistant and air-permeable nonwoven fabric, the inner sheet 6 and the outer sheet 7 may be formed of an air-permeable nonwoven fabric, the leak-barrier sheet 14 may be formed of a moisture-pervious plastic film, and the liquid-absorbent core 16 may be formed from a mixture of fluff pulp and super-absorbent polymer particles, all of these stock materials being conventionally used in the relevant technical field. The first, second, third and fourth joint zones may be formed using the techniques conventionally employed in the relevant technical field, e. g. , adhesives, thermal or ultrasonic bonding means.
While the diaper 1 has been exemplarily described on the basis of the pullon-pant type diaper 1, the present invention is applicable also to the open-type diaper having the front and rear waist regions joined together immediately before the diaper is put on the wearer.

### <Second Embodiment>

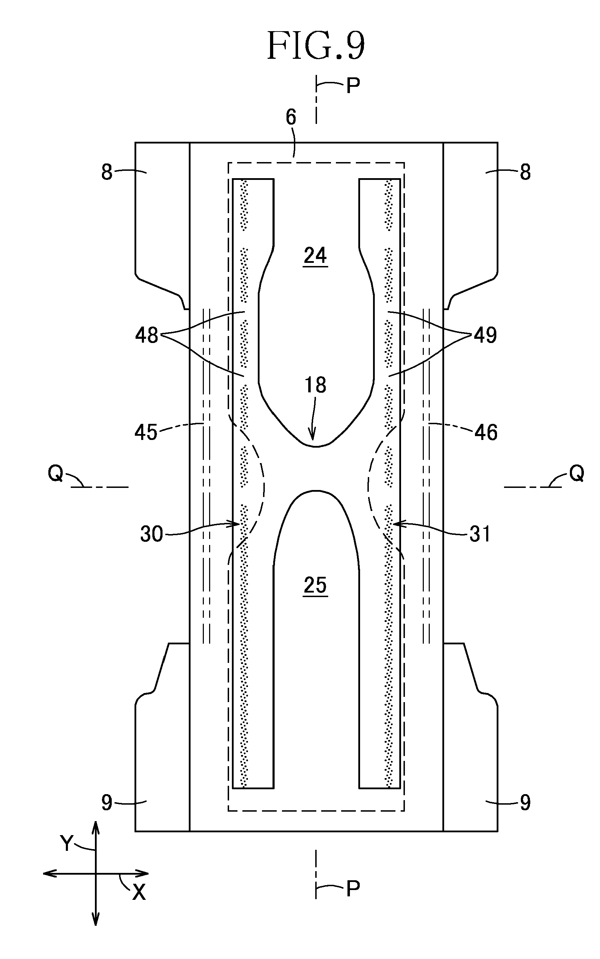

Fig. 9 is a plan view of a flatly developed diaper 1 according to a second embodiment wherein the leak-barrier cuffs 35, 36 are not illustrated for convenience of illustration. In this embodiment, the components similar to those in the first embodiment are designated by the reference numerals similar to those used in the first embodiment and detailed description of these components will be not repetitively described. This embodiment is characterized by fluid-communicating pores 48, 49 provided in the first joint zones 30, 31 for fluid-communication between the interior and the exterior of these joint zones 30, 31. A plurality of such fluid-communicating pores 48, 49 is intermittently formed at least in regions defined aside from the transverse center line Q-Q toward the front waist region 3. These fluid-communicating pores 48, 49 allows urine guided through the front guide passage to move outward beyond the first joint zones 30, 31 in the transverse direction X and then to be absorbed by the liquid-absorbent structure 15 also outside these first joint zones 30, 31 also. In this way, the area over which urine comes in direct contact with the liquid-absorbent structure 15 is enlarged and a rate of urine absorption is correspondingly accelerated, resulting in an improved preventive effect against leakage of urine.

The fluid-communicating pores 48, 49 may be formed in the regions defined aside from the transverse center line Q-Q toward the front waist region 3 and such an arrangement is effective to reduce a possibility of urine leak because urine is smoothly guided through the front guide passage 24 formed on the side of the front waist region 3 to the liquid-absorbent structure 15. However, it is not essential to form these fluid-communicating pores 48, 49 only on in the front waist region 3 but it is also possible to form them in the crotch region 5 also.
The number as well as the size of the fluid-communicating pores 48, 49 to be formed may be appropriately varied depending on a particular size of the diaper 1.

## Claims

1. An absorbent article comprising a chassis having longitudinal direction and transverse direction, sides facing the skin of a wearer and a garment worn by the wearer, respectively, a front waist region, a rear waist region and a crotch region and a liquid-absorbent structure placed at least in said crotch region, a separate sheet provided on said side facing the skin of the wearer of said chassis so that said separate sheet may be spaced apart from said crotch region, and leg elastic members attached under tension in said longitudinal direction so as to extend across said crotch region in said longitudinal direction, said absorbent article being **characterized in that**:
said separate sheet comprises a pair of lateral regions opposed to each other in said transverse direction and extending in said longitudinal direction, a middle region lying between said lateral regions so as to connect said lateral regions to each other, guide passages adapted to ensure fluid-communication between said side facing the skin of the wearer and the side of said chassis, and joint zones used to join said lateral regions to said chassis, wherein said separate sheet is elasticized in said longitudinal direction at least in said crotch region and said joint zones are formed so as to overlap said liquid-absorbent structure;
said leg elastic members are provided outside said liquid-absorbent structure as viewed in said transverse direction; and
a void space is formed between said separate sheet and said liquid-absorbent structure and at least said middle region comes in contact with the skin of the wearer as said liquid-absorbent structure is curved so that front and rear ends thereof opposed in said longitudinal direction and extending in said transverse direction face each other in said absorbent article put on the wearer.

2. The absorbent article defined by Claim 1 wherein said joint zones are arranged so as to extend in said longitudinal direction at least in said crotch region.

3. The absorbent article defined by Claim 1 or 2 wherein said lateral regions of said separate sheet are formed with folded zones by folding back said lateral regions toward the side of said chassis along fold lines extending in said longitudinal direction and said joint zones are formed between each of said folded zones and chassis.

4. The absorbent article defined by any one of Claims 1 through 3 wherein said joint zones include fluid-communicating pores for fluid-communication between the interior and the exterior of said joint zones as viewed in said transverse direction in regions aside from a transverse center line bisecting a dimension of said chassis in said longitudinal direction toward said front waist region.

5. The absorbent article defined by any one of Claims 1 through 4 wherein:
said chassis further comprises a pair of leak-barrier cuffs extending in said longitudinal direction on said side facing the skin of the wearer; and
each of said leak-barrier cuffs includes a proximal side edge fixed to said chassis and a free side edge adapted to be spaced from said chassis, said proximal side edge being provided with said leg elastic members attached thereto under tension.

6. The absorbent article defined by any one of Claims 1 through 5 wherein said leg elastic members extend substantially in parallel to the lateral regions of said separate sheet.

7. The absorbent article defined by any one of Claims 1 through 6 wherein:
said leg elastic members extend from respective boundary points between said front waist region and said crotch region to respective boundary points between said rear waist region and said crotch region;
respective opposite ends of said leg elastic members lie outside said liquid-absorbent structure as viewed in said transverse direction; and
said joint zones overlap said liquid-absorbent structure at positions corresponding to said respective opposite ends.

8. The absorbent article defined by any one of Claims 1 through 7, wherein:
said separate sheet further comprises a pair of separate sheet elastic members, and said separate sheet elastic members are attached under tension at least along lateral edges of said guide passages.
